# EUROPEAN PATENT APPLICATION

(11) **EP 4 390 946 A1**
(43) Date of publication of application: **26.06.2024**
(21) Application number: 22214801.7
(22) Date of filing: 20.12.2022
(51) Int. Cl.: G16H 10/60, G16H 40/63

(54) **HEALTHCARE APPARATUS FOR DISPLAYING EHR DATA**

(71) Applicant: Koninklijke Philips N.V., 5656 AG Eindhoven (NL)
(72) Inventor: SERLIE, Iwo, Eindhoven (NL); BULUT, Murtaza, Eindhoven (NL); BOUWMAN, Wilbert, 5656AG Eindhoven (NL); RASMIJN, Anita, Eindhoven (NL)
(74) Representative: Philips Intellectual Property & Standards

(57) **Abstract**

According to an aspect, there is provided a healthcare apparatus comprising: a memory comprising instruction data representing a set of instructions; and a processor configured to communicate with the memory and to execute the set of instructions. The set of instructions, when executed by the processor, cause the processor to: i) receive a first user input from a user input device, the first user input indicating a first zoom command, the first zoom command being indicated with respect to a first portion of a display in which first data from an electronic healthcare record, EHR is displayed; ii) retrieve second data that is semantically linked to the first data; and iii) in response to receiving the first user input, instruct the display to display the second data in a second portion of the display.

## Description

### FIELD OF THE INVENTION

Embodiments herein relate to healthcare apparatus, particularly but non-exclusively, embodiments herein relate to the retrieval of information from an electronic healthcare record (EHR).

### BACKGROUND OF THE INVENTION

The disclosure herein relates to the field of clinical decision support (CDS) and to the retrieval of medical information in electronic healthcare records (EHR). EHRs are digital versions of the paper records traditionally kept by hospitals related to a patient's care and treatment. EHRs may comprise the medical history and treatment history for a patient. They may further comprise diagnoses, medications, treatment plans, immunisation history, allergies, radiology images, and laboratory test results as well as any other information or notes made by a clinician or carer of the patient.

Healthcare professionals (HCPs) need the right information at the right time to make informed decisions. This generally entails the collection of significant amounts of patient data. In addition, HCPs require different information depending on their roles.

Generally, current tools to retrieve patient information from an EHR may be configured to retrieve particular data entries or types of data entries, based on the user's authorization, meaning that it is possible to retrieve only the data that is relevant to each HCP role.

Moreover, professionals and non-medical professionals (including the patients themselves) have different data requirements. A professional is trained to use medical terms and local jargon. An amateur may need more explanation and jargon close to their own education. Note that a specialized HCP may be a professional in one domain and a layman in another.

It would be beneficial to have improved data retrieval solutions to enable medical data from EHRs to be retrieved with predictable behaviour for different users.

### SUMMARY OF THE INVENTION

Typically, a hierarchical model of progressive data disclosure is implemented in an EHR retrieval system, in order to enable a HCP to explore patient information. Data is delivered in small, manageable bits. Based on initial data, a user can decide to retrieve more data that is related to the initial data. It is an object of embodiments herein to improve upon data retrieval for HCPs from EHRs.

According to a first aspect, there is provided a healthcare apparatus comprising: a memory comprising instruction data representing a set of instructions and a processor configured to communicate with the memory and to execute the set of instructions. The set of instructions, when executed by the processor, cause the processor to: i) receive a first user input from a user input device, the first user input indicating a first zoom command, the first zoom command being indicated with respect to a first portion of a display in which first data from an electronic healthcare record, EHR is displayed; ii) retrieve second data that is semantically linked to the first data; and iii) in response to receiving the first user input, instruct the display to display the second data in a second portion of the display.

According to a second aspect there is a computer implemented method performed by a healthcare apparatus. The method comprises: i) receiving a first user input from a user input device, the first user input indicating a first zoom command, the first zoom command being indicated with respect to a first portion of a display in which first data from an electronic healthcare record, EHR is displayed; ii) retrieving second data that is semantically linked to the first data; and iii) in response to receiving the first user input, instructing the display to display the second data in a second portion of the display.

According to a third aspect there is a computer program product comprising a computer readable medium, the computer readable medium having computer readable code embodied therein, the computer readable code being configured such that, on execution by a suitable computer or processor, the computer or processor is caused to perform the method of the second aspect.

Thus, the apparatus and methods herein facilitate what may be considered as specialized semantic zooming of medical information, whereby receipt of a zoom command from a user initiates retrieval of further semantically linked information from the EHR, or other data source (related to the first data) in the region of the zoom command. Thus, in this manner, a HCP can zoom-in and out of a medical record to receive further information related to the first data. The method may further improve the time it takes to access relevant data, and can ensure that more relevant data is retrieved.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an apparatus according to some embodiments;
Fig. 2 shows a method according to some embodiments;
Fig. 3 shows an example display according to some embodiments herein; and
Fig. 4 shows an example system according to some embodiments herein.

### DETAILED DESCRIPTION OF EMBODIMENTS

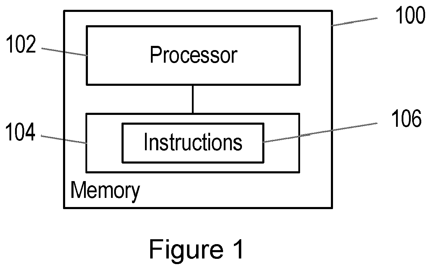

Turning now to Fig. 1 in some embodiments there is a healthcare apparatus 100 for use in healthcare setting, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 200 as described below.

Embodiments of the healthcare apparatus 100 may be for use in retrieving information from an EHR. More specifically, the set of instructions, when executed by the processor, cause the processor to: i) receive a first user input from a user input device, the first user input indicating a first zoom command, the first zoom command being indicated with respect to a first portion of a display in which first data from an electronic healthcare record, EHR is displayed; ii) retrieve second data that is semantically linked to the first data; and iii) in response to receiving the first user input, instruct the display to display the second data in a second portion of the display.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the EHR, or a portion thereof, the zoom command, the first portion of the display (or the location thereof), the second data and/or any other information or data received, calculated or determined by the processor 102 of the apparatus 100 or from any interfaces, memories or devices that are external to the apparatus 100. The processor 102 may be configured to control the memory 104 to store the EHR, or a portion thereof, the zoom command, the first portion of the display (or the location thereof), the second data and/or the any other information.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may comprise a battery or other power supply for powering the apparatus 100 or means for connecting the apparatus 100 to a mains power supply.

As a further example, the apparatus 100 may further comprise a display. For example, the display referred to in steps i) and iii) may form part of the apparatus 100. Generally, a display may comprise, for example, a computer screen, a screen on a mobile phone, a screen on a tablet, or a screen associated with a hospital monitor. In some examples, a display may be a three-dimensional display, virtual reality display or any other display mechanism capable of displaying data from an EHR.

It will be appreciated however that the display does not have to be part of the apparatus 100, for example, the display may be separate from the apparatus 100. As such, in some embodiments, the apparatus 100 may communicate with the display via a wired or wireless connection.

The apparatus 100 may further comprise a user input device. For example, the user input device referred to in step i) may form part of the apparatus 100. For example, in some embodiments, the display is a touch screen display and the user input device may be the touch sensitive aspects of the touch screen display. In such embodiments, the first zoom command may be indicated by the user using known touch motions. For example a "zoom-in" command may be indicated by placing two fingers on the screen in the first portion of the display and spreading the fingers apart. A "zoom-out" command may be indicated by the inverse motion, e.g. by placing two fingers of the screen and bringing them closer together. As another example, a zoom command may be input by touching a screen more than a threshold number of times within a certain time interval (e.g. a zoom command may be input using a double, or triple click), or through the use of an on-screen scroll bar or similar. These are merely examples however, and the skilled person will be a familiar with touch-screen displays and motions performed thereon to indicate a zoom command.

In other embodiments, the user input device is, for example, a keyboard, mouse or other input device that enables a user to interact with the apparatus, for example, to provide the first zoom command. As an example, a wheel on a mouse can be used to indicate a "zoom-in" command, for example, an upward motion may indicate a zoom-in command and a downward motion may indicate a "zoom-out" command. The skilled person will appreciate that these are merely examples however and that a zoom command may be indicated in other ways to those described herein.

It will be appreciated however that the user input device does not have to be part of the apparatus 100. For example, the user input device may be separate from the apparatus 100. As such, in some embodiments, the apparatus 100 may communicate with the user input device via a wired or wireless connection.

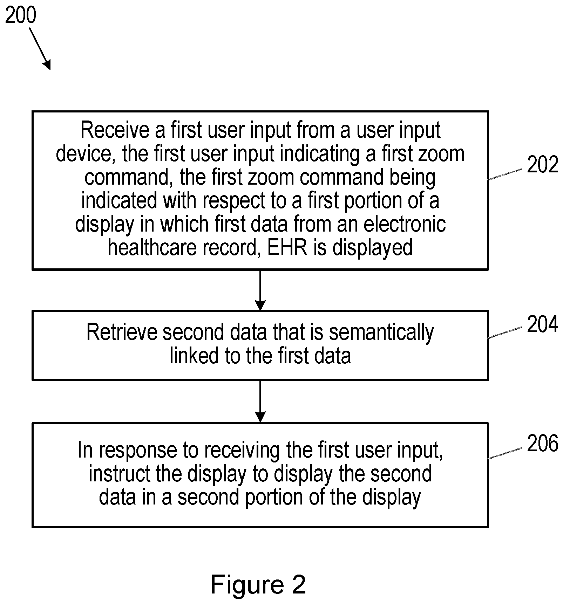

Turning to Fig. 2, there is a computer implemented method 200. The computer implemented method 200 may be performed by a healthcare apparatus such as healthcare apparatus 100 above.

Briefly, in a first step 202, the method 200 comprises: i) receiving 202 a first user input from a user input device, the first user input indicating a first zoom command, the first zoom command being indicated with respect to a first portion of a display in which first data from an electronic healthcare record, EHR is displayed. In a second step 204 the method comprises ii) retrieving 204 second data that is semantically linked to the first data. In a third step 206 the method 200 comprises: iii) in response to receiving the first user input, instructing the display to display the second data in a second portion of the display.

In more detail, preceding step i), the method 200 may comprise instructing the display to display the first data from the EHR. In this sense, the first data may be a part of, (or a portion of) the full information in the EHR. As described above, an electronic healthcare records (EHR) is a digital version of the paper records traditionally kept by hospitals related to a patient's care and treatment. EHRs may comprise the medical history and treatment history for a patient. They may further comprise diagnoses, medications, treatment plans, immunisation history, allergies, radiology images, and laboratory test results as well as any other information or notes made by a clinician or carer of the patient.

Generally, an EHR may comprise other parameters to those described above, for example, monitoring data and signals, vital sign information, algorithm output, billing and financial information, scheduling, resource management. There may be different dashboards showing metrics such as operational status, clinical status, and/or any other key performance indicators.

The data in an EHR may be dynamic, for example, it may be updated in a regular manner. For example, additional new data may be added, and/or updates to the existing data may be made. As an example, if vital sign information is taken every 15 minutes, the new vital sign information may be stored in the EHR.

The data stored in the EHR can be stored in an automated manner (e.g. via device to device communication protocols), manually by humans, or in a hybrid manner, e.g. where humans verify the data before storing it in the EHR.

The first data may be displayed along with other data, either from the EHR, or from other sources.

A user of the apparatus 100, such as a doctor, clinician, radiologist, nurse, other HCP, the patient themselves, or any other user may view the displayed first data and provide the first user input. The first user input may be received from the user input device. User input devices were described above with respect to the apparatus 100 and the detail therein will be understood to apply equally to the method 200. The first user input may be received e.g. via a wired or wireless connection. In other embodiments where the user input device forms part of the apparatus 100, the first user input may be received directly from the user input device.

The first user input indicates the first zoom command. For example the first user input may indicate a type of zoom command, e.g. a "zoom-in" command, or a "zoom-out" command. The user may indicate the first zoom command using the user input device as described above with respect to the apparatus 100 and the detail therein will be understood to apply equally to the method 200.

The zoom command is indicated with respect to a first portion of a display. In other words, the user provides an indication to zoom-in or to zoom-out on the first data displayed on the display.

Generally, the display may be divided into a plurality of portions, for example, there may be different portions associated with each piece of data displayed on the display, or associated with different groups of data on the display. The zoom command may be executed differently in each portion.

It will be appreciated however that the display doesn't necessarily have to be divided into different portions. Thus, in some embodiments, the first portion of the display (as referred to herein) may refer to the entirety of the display or graphical user interface (GUI).

In response to receiving the first user input indicating the zoom command, the method 200 moves to step ii) and retrieving second data that is semantically linked to the first data.

In this context, the second data is semantically linked to the first data if there is a meaningful or logical connection between the second data and the first data. In other words the second data is related to the first data in some manner. For example, the second data may be semantically linked to the first data if the second data relates to the same, or similar subject or topic as the first data.

For example, if the first data indicates a medical condition, then the second data may be semantically linked to the first data in the sense that it provides more detailed (or conversely more general) information about the medical condition.

As another example, if the first data indicates a course of action or treatment, then the second data may be semantically linked to the first data in the sense that it provides more detailed (or conversely more general) information about the course of action or treatment.

As another example, the first data may relate to a medical condition referred to in the EHR and the second data may relate to a treatment given for the medical condition. In another example, the first data relates to a medical condition referred to in the EHR and the second data relates to further information related to said medical condition. In another example, the first data relates to a type of medication referred to in the EHR and the second data relates to specific medications (or brands of the same medication) within said type.

As another example, if the first data is a medical image (such as a CT scan, an x-ray scan, an MRI scan etc.), then the second data may be semantically linked to the first data in the sense that it provides more information on particular structures, lesions or other features detected in the medical image.

Other examples of first data and second data include, but are not limited to:
Vital sign measurements and further related vital signs
Prediction algorithm output and details explaining the output, e.g. a list of most significant inputs that contributed to the output
Appointment in calendar and further details, including location, staff, resources needed, etc.
Surgery planning and surgery documentation
Clinical note and a related clinical notes
Portion (text) of a clinical note and a related clinical notes
Clinical protocol (or procedure) and more information about the protocol
Physical order entry and more information/details about the order
Billing and more detailed billing information
Patient administration and more detailed patient administration
Patient registration and more detailed patient registration
Patient overview and individual patient details
Operational dashboard and more details about specific elements
An example sequence of four types of data that may be displayed in a sequence of successive "zoom in" commands is: Disease --> disease subtype --> mechanism of disease --> genetic information

Note that in the examples above, the second data may relate to the particular patient e.g. the second data may be taken from the EHR. Alternatively or additionally, the second data may be taken from one or more other sources. For example, medical guidelines, medical journals, medical texts, a medical resource database, medical library, medical reference tools, clinical decision support systems, or any other source. References by government bodies may also be used, for example, resources from the Food and Drugs Administration (FDA).

It is further noted that the first data and the second data may be different types, for example, the first data may be in text format and the second data may be in an image format (e.g. such as a medical image) or vice versa. Thus the first data may be a first data type and the second data may be a second data type that is different to the first data type. Examples of data types include but are not limited to: images, text strings, animations, data in a tabular format, data presented in a graphical format, a hyperlink, simulation data, audio data, 3D data or images, video data, or any other data type.

In some embodiments, the second data is more detailed than the first data if the first user input indicates a zoom-in command. In some embodiments, the second data is less detailed than the first data, if the first user input comprises a zoom-out command. Thus in this manner, the user can zoom-in and out to see more, or less detailed information respectively. According to the methods herein, data is retrieved in a logical and intuitive manner in response to a zoom command.

The second data may be retrieved in different ways. For example, the second data can be logically connected to the first data as part of a hierarchical data structure in the EHR. An example hierarchical data structure is an XML data structure whereby data can be stored in a sequence of hierarchical layers. Another example of a hierarchical data structure is the Flexible Image Transport System (FITS) header structure, which is frequently used to store radiological images.

Another example of a hierarchical structure is the Fast Healthcare Interoperability Resources standard HL7FHIR. In such an example, the structure within the standard may determine the manner in which the data is linked in a zoom command. An example may be summarised as follows: food allergy: category (food | medication | environment | biologic ) --> zoom in --> type (allergy | intolerance - Underlying mechanism). See the HL7 FHIR documentation, section 9.1 entitled: "Resource Allergy Intolerance", for more information.

An example of a hierarchical structure to represent relationships between different medical terms and concepts is to use a coding mechanism showing how different concepts hierarchically link to each other. For example, for medications, a structure similar to the Anatomical Therapeutic Chemical Classification System (ATC medication codes) may be used.
Generally, in embodiments where the data is in a hierarchical data structure, the structure can be learned from the data, or it can be pre-determined, for example, by referring to the EMR architecture, and data transfer, management and interoperability standards.

As such, the second data can be in a neighbouring level of the hierarchical structure to the first data. In other words, the user may be able to zoom-in to see data that is further down the hierarchical data structure and zoom-out to see data that is higher up in the hierarchical data structure.

In other embodiments, receipt of the zoom command may execute a computational function to retrieve the second data that is semantically linked to the first data. As an example, the first data may be one of the inputs to such a computational function, which is linked to the first portion of the display. In such an example, the zoom action may cause the computational function to be executed and a function output to be generated (it may be the case that the function was executed and output generated beforehand, for instance if the function is executed with set frequency or based on other triggers). In such embodiments, the second data is then determined according to the function output.

Inputs to such a function include but are not limited to the following examples: the EHR, the user identity, information from a profile of the user, and the data displayed in the first portion of the display.

The function that is executed may be pre-determined, but different executions may result in different outputs because it is possible that:
- the first data changes
- it makes use of other inputs, in addition to first data, and if these inputs change, the function output would change. Example, of other inputs in addition to first data are time, other EHR data, or user related input (previous zoom actions, profile).
- the function changes, because it is stochastic (and not deterministic), or because the function algorithm has been retrained/updated.

As noted above, the EHR may change due to the potentially dynamic nature of the information therein.

Thus, the function output may be thought of as a current version of the meaning/interpretation of the first data, and if this meaning changes the semantically linked data (second data) may also change.

As an example, a computational function as described above may use natural language processing or machine learning to determine the meaning of the first data. An example of a manner in which semantic meaning can be determined from clinical data is described in the paper by Kersloot, M.G., van Putten, F.J.P., Abu-Hanna, A. et al. entitled: "Natural language processing algorithms for mapping clinical text fragments onto ontology concepts: a systematic review and recommendations for future studies". J Biomed Semant 11, 14 (2020). https://doi.org/10.1186/s13326-020-00231-z. The concepts in this paper may be used to determine semantically linked words that may be used as search strings or input parameters to a data query that can be used to retrieve the second data.

Some further example approaches that can be used to compute semantic meaning and semantic links (e.g. that can be used to determine second data that is semantically linked to the first data) are the following:
Natural language processing (NLP): NLP algorithms can be trained to analyze the structure and meaning of sentences, identify relationship between words and phrases, and extract relevant information from text. For example, using disease medical literature for training, the relationship between different words and phrases can be learned. This relationship can be later used, to determine the output of the zooming function. For instance, it can be learned that certain medications are mentioned in the text that described a certain disease. Based on this information, the disease and medication names can be assumed to be semantically linked.

Another example is to look at the links between different words within the same sentence or within a set distance from each other in the text. Then it can be assumed that words/phrases that are within certain distance from each other are semantically linked.

Using natural language algorithms for semantic analysis is a well-developed field, and the skilled person will be familiar with appropriate techniques that may be used. Some further references are: Chintalapudi N. et.al. (2021) "Text mining with sentiment analysis on seafarers'medical documents"; Feldman R., Regev Y. et.al. (2004), "Mining the biomedical literature using semantic analysis and natural language processing techniques" and Velupillai S. et.al. (2015), "Recent Advances in Clinical Natural Language Processing in Support of Semantic Analysis"; 10(1):183-93. doi: 10.15265/IY-2015-009. Examples of algorithms types are the following.

Word Embedding Processes: methods to represent words as vectors (word embedding algorithms), such as word2vec and GloVe, where words with similar meanings are located close to each other. Then these distances can be used to identify semantic relationships between words.

Semantic network: this a graphical representation of the meaning of words or phrases in a given text. It is represented as a network of nodes and edges, where the nodes are concepts or entities, and edges represent the relations between them.

Semantic role labelling (SRL): here the roles of different words or phrases are identified in an automated manner. For examples some words can be labelled as "agents", some like "action", and so on.

When using machine learning algorithms (such as NLP) to find semantic links in medical data, one approach is to use large datasets of medical data. The learning can be performed based on supervised or unsupervised manner.

When performed in unsupervised manner the relations can be learned in an automated manner from the text. For example, this is possible because linked words will appears in close proximity to each other. Using the section division, chapter division, paper clusters/categories classifications, etc. used in the medical literature, the relationship of the word to the concepts can be learned. K-means clustering and hierarchical clustering are example techniques that can be used.

Another example is to use annotated data to learn semantic links. In this example, the typical use of EMR can be used to determine and learn the relations between different types of EMR data and fields. For example, usage data indicating the origin of clicks, and their resulting destinations and the content of the origin and destination can be collected. Such information can be used as labels for connections between different EMR fields. In other words, by tracking how clinicians use the EMR, if they go from data1 to data2 fields, and the content of these data fields, and by using such information as ground truth while training machine learnings semantic links as applicable to human users can be learned. For example, it can be assumed that data1 and data2 data or data fields are linked. Using this information, the trained machine leaning algorithms can predict the linked data or data fields, given an input data or data field. Support vector machines (SVMs) and decision trees are example methods that can be used.

Other techniques include but are not limited to: deep learning algorithms, such as convolutional neural networks, recurrent neural networks, and long short-term memory (LSTM) networks.

Latent Dirichlet allocation (LDA): a probabilistic generative model that can be used to identify topics in a corpus of data. For example, semantic relations such as synonyms and antonyms can be identified.

Latent semantic analysis (LSA): mathematical technique that uses singular value decomposition (SVD) to identify the relationships between words and concepts.

Word2vec: deep learning algorithm for vector representation of words. These vector representations can be used semantic relationships, for example similarity, relatedness, etc. See: Mikolov, Tomas; et al. (2013). "Efficient Estimation of Word Representations in Vector Space". arXiv:1301.3781.

GloVe is another algorithm similar to Word2vec. See: Jeffrey Pennington, Richard Socher, and Christopher Manning. 2014. GloVe: Global Vectors for Word Representation. In Proceedings of the 2014 Conference on Empirical Methods in Natural Language Processing (EMNLP), pages 1532-1543, Doha, Qatar. Association for Computational Linguistics.

BERT: this is transformer based deep learning algorithm. It is very powerful, and it has many variations which can be used to identify semantic relationships. See: "BERT: Pre-training of Deep Bidirectional Transformers for Language Understanding" by Jacob Devlin, Ming-Wei Chang, Kenton Lee, Kristina Toutanova, arXiv:1810.04805.

Turning back now to the method 200, after step ii) the method moves to step iii) where in response to receiving the first user input, the method 200 comprises instructing 206 the display to display the second data in a second portion of the display.

The second portion of the display may be the same as the first portion of the display. In other words, step 206 may comprise instructing the display to display the second data in place of (e.g instead of, or in the same region as) the first data. In some embodiments, the second portion of the display is at least partially overlapping with the first portion of the display. As a result, step 206 may comprise instructing the display to display the second data in place of the first data.

In other embodiments, the zoom command causes a new window, or graphical user interface (GUI) to appear. In such an embodiment, the zoom command may cause the new window to pop-out and display the second data. In other words, the first portion of the display may be in (e.g. correspond to) a first GUI and the second portion of the display may be in (e.g. correspond to) a second GUI. The processor may cause the second GUI to be initiated on the display in response to receiving the first user input.

Thus, in this way, a zoom command initiated on first data displayed on a display can be used to retrieve and display second data from, or related to, an EHR that is semantically related to the first data.

Further zoom commands may subsequently be received from the user and processed according to the method 200. For example, if a second zoom command is received that indicates zooming in an opposite direction to the first zoom command (e.g. if the first zoom command is a zoom-in command and the second zoom command is a zoom out command, or vice versa) then the second zoom command may (at least partially) reverse the first zoom command.

Put another way, the method 200 may further comprise receiving a second user input from the user input device, the second user input indicating a second zoom command with respect to the first portion of the display. The second zoom command may at least partially reverse the first zoom command. As such, the method 200 may further comprise instructing the display to re-display the first data in the first portion of the display.

In embodiments where the first and second data is part of a hierarchical structure, a zoom command in a first direction may result in data being displayed from a higher level in the hierarchy whereas a zoom command received in a second, opposite direction may result in data being displayed from a lower level in the hierarchy.

In embodiments, where the zoom command is linked to a computational function, a second zoom command in the opposite direction this may be interpreted as calculation of an inverse of the computational function. As another example, the computational function could be a different function, where the second data is one of the inputs. In another example, it could be a function where both first and second data are inputs. In other words, all the previous links that were used to arrive to the current data fields can be kept in memory for the usage session of the EMR, and the zooming/unzooming history can be used as one of the inputs for the computational function. Making use of the historical zooming actions (previous zooms) is also one of the reasons why zooming at the same region at different time points can result in different outputs.

In some embodiments, in response to receiving the second zoom command, the method 200 comprises retrieving third data that is semantically linked to the second data, and instructing the display to display the third data in a third portion of the display. In such a scenario, the third data may be different to the first data, if, since the processor performed step ii): there has been a change in the first data; there has been a change in the second data; or if the third data has become available (e.g. since the processor performed step ii)), and as a result, the third data is more highly semantically linked to the second data compared to the first data.

In other words, in this embodiment, if the second zoom command is an inverse zoom command to the first zoom command (e.g. if the second zoom command is in the opposite direction to the first zoom command) then this will not necessarily result in the first data being redisplayed if information that is more semantically relevant to the second data (compared to the first data) is now become available.

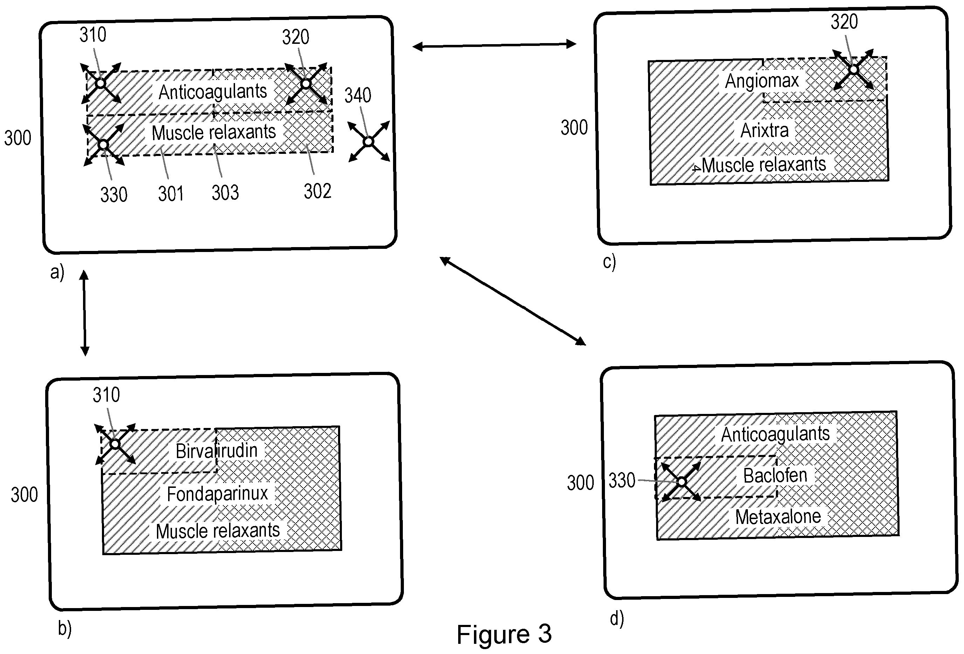

Turning now to Fig. 3 which shows an example according to some embodiments herein. In this example, a display 300 (or viewport) with medical concepts is shown in Fig. 3(a), where different regions (301, 302) indicated by the dashed and hatched regions are recognized per medical concept. In this example, each region is associated with a zoom function for that specific region. Zooming on the first region (301, not mouse position) using a mouse at position 310 activates a first zoom function (otherwise referred to herein as a computational function), resulting in the display shown in Fig. 3b. Zooming on the second region (302) e.g. at mouse position 320 activates a second zoom function, resulting in the display shown in Fig. 3c. Zooming on the background (340) activates a third zoom function, resulting in the display shown in Fig. 3d. Take for example, zooming medication data. The first zoom function may provide generic names for professionals, the second zoom function may provide brand names for laymen, and the third zoom function may affect the font size of text.

Thus, Fig. 3 illustrates a system for zooming semantic information in a viewport, wherein the information comprises a multitude of regions (e.g. regions 301, 302), each region having a zoom function associated with that region.

Take, for example, anticoagulants as displayed in Fig. 3a. When zooming with the mouse cursor on the left side of the concept at 310, the first zoom function may show generic names of actual medications (e.g., Bivalirudin as illustrated in Fig. 3b), the region for muscle relaxants remains unchanged. When zooming with the mouse cursor on the right side of the concept at 320, the second zoom function may show brand names of the same medication (e.g., Angiomax as illustrated in Fig. 3c). Note that, as described above, the regions may be configured such that the zoom-in and zoom-out commands support inverse operations.

Furthermore, the system may be extended to support a plurality of zoom functions for one region, where a specific zoom function is associated with the semantic type of the underlying medical concept. Take, for example, zoom functions for zooming anatomical data in addition to aforementioned zoom functions for medication data. The first zoom function may show Latin names for anatomy (e.g., hepaticus) and the second zoom function may show translated anatomical names for laymen (e.g., liver). The proposed concept thus provides a single solution for exploring medical data, recognizing the needs of different users, allowing users to decide for themselves what level of information they need, while minimizing data shown in a viewport.

Note that regions do not have to be predefined and do not have to be part of the source data. The regions can be dynamically obtained based on the underlying meaning of the data. For example, by grouping the displayed content according to meaning (e.g. using the NLP method described above) and splitting the display into different portions accordingly. The system can dynamically link content to particular zoom functions. Consequently, configuration can be minimal, and medical data can be zoomed from a database without containing information of zoom regions.

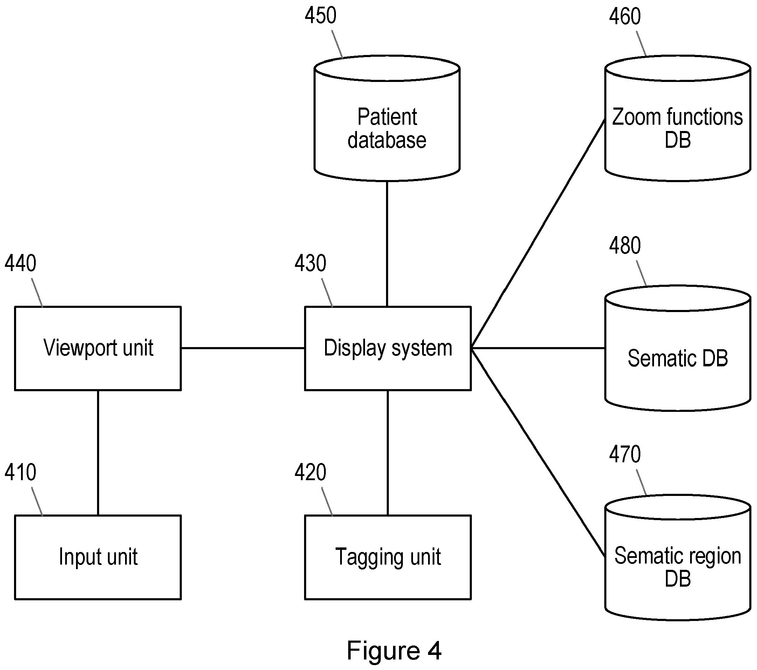

Turning now to Fig. 4 which shows a plurality of computing modules 400 that can be used to implement the method 200. The modules 400 may be implemented in an apparatus such as the apparatus 100 described above.

In this embodiment, there is a display system 430 that is configured to display medical concepts such as an EHR from a patient database 450 onto a viewport unit 440.

The system furthermore comprises a tagging unit 420 that is configured to compose multiple display regions for medical concepts that are displayed in the viewport unit. Firstly, it recognizes the type of medical concept using a semantic DB 480. For example, "Anticoagulants" is of type medication.

Medication categories and types (including brands) may be available in the hospital system and NLP, or any of the other techniques described above, can be used to create semantic DB 480, as described above. Also, as described above, the FHIR standard, or other data structure may be used to link data in the semantic database. As an example, the output of the NLP algorithms described can be used to show the relationships between different words and concepts. For example if words (or group of words) are represented as numerical vectors, the semantic DB 480 can contain this information. Also, the relation between different words/concepts can be represented as decision trees, or connected graphs.

As another example, the semantic database may follow a structure similar to the Unified Medical Language System (UMLS), see UMLSO Reference Manual; Bethesda (MD): National Library of Medicine (US); 2009. As an example, semantic DB 480 can follow a structure similar to UMLS, but the relations can be learned, updated automatically taking into account updated patient data, updated protocol and workflow execution data, clinician and organizational profile, for example,

Secondly, the system receives region definitions from a semantic region DB 470 for a specific type. Semantic region DB 470 may comprise rules/code that determine the user interface or user interaction related aspects. These are aspects linked to how the active regions (regions with zoom functionality) are determined and indicated to the user. These may be based on pre-determined rules. For example, a rule may be: "use the left 20% as left region, and 20% of right as right region". Another example of a rule may be: "use the outmost pixels of the word to determine a boundary for the word, and use pixels that are within 20 pixel distance from the boundary as the active region". It will be appreciated however that these are merely examples, and that the semantic region DB may comprise different rules or combinations of rules to those described herein.

In some embodiments, the regions can be automatically updated, learned or determined based e.g. on tracked interactions of human users with the EHR system. They can be also learned or copied from other systems that show similar types of data.

Depending on the information type displayed on the EMR screen (e.g., billing, patient data, scheduling data, etc.) the definitions for active regions may be different.

In some cases, the definition of regions may be determined or influenced by the number, or type, of the links assigned to a particular data entry. For example, an increase in the number of hierarchical links may result in an increase in the number of active regions, (and/or a decrease in the size of the active regions).

Each region is identified with a "left" or "right" position indicator. Thirdly, it applies the region definitions to a specific medical concept (e.g., Anticoagulants, see Fig. 3a).

The semantic display system 430 assigns a zoom function from a zoom function database 460 to each composed display region.

The zoom functions (otherwise referred to herein as zoom commands) are then received for a specific medical concept type such as medication. A zoom function is then associated with a specific region in the semantic region database 470, where a zoom function is tagged with a position indicator (e.g., "left", "right").

The system comprises an input unit 410 for selecting a region from a plurality of regions on a viewport 440. For example, a user selected a left region on Anticoagulants.

The system 430 is configured to apply the selected zoom function to the selected medical concept. For example, a zoom function is defined to reveal all brand names underlying medication. To accomplish this, a semantic database 480 may contain various known brand names (e.g., Angomax). Additionally, the database may comprise a relation from brand names to the medication type. The list of filtered brand names is intersected with data in a patient database. The intersection is displayed in a viewport unit 440 (see Fig. 4b).

The implementation may be enhanced in multiple ways.
- The region position may be extended with additional region indicators such as top, bottom, and middle positions.
- Not all data sources are structured and codified. The system may recognize the semantics from a text on the display in an automated manner. This enables semantic zooming on sources that traditionally may not have been intended to be semantically zoomed. As another example, an external document may function as a table of contents for patient information such as an EHR. As such, this allows progressive disclosure of patient information steered by a document that is not part of the system.
- A person skilled in the art will be able to apply the method to other medical phrases as well as isolated medical concepts.
- The word/concept/content of document on which the mouse points to can be recognized, and the zooming function can be associated to displaying other words linked to the corresponding word. For instance, for a given medication, other medications with the same active ingredient can be displayed or hidden depending on zooming-in and zooming-out actions respectively.
- As another example, the zooming action can be associated to show or hide the content of a link. The link can be a hyperlink to a webpage for example, or QR code. In which case, zooming when hoovering over the link will cause the content associated with the link to be shown with increasing detail and amount, while zooming out will decrease the amount of the information.
- The system may be enhanced to recognize parts of words. For example, enabling to zoom in on parts of medication names. Many treatments are using Monoclonal antibodies•(MABs). When zooming in on infliximab will result all monoclonal antibodies or towards all MAB treatments related towards the disease of the patient.
- The system may be enhanced to recognize the way information is shown on the screen. For example font style, specific characters being used or specific pixel combinations of graphics, allowing multiple zoom functions based on the way that information is shown on a display.

Thus, in this manner, semantic zooming may be used to search and retrieve information in an EHR in a more intuitive and comprehensive manner.

Turning now to other embodiments, in some embodiments, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A healthcare apparatus comprising:
a memory comprising instruction data representing a set of instructions; and
a processor configured to communicate with the memory and to execute the set of instructions, wherein the set of instructions, when executed by the processor, cause the processor to:
i) receive a first user input from a user input device, the first user input indicating a first zoom command, the first zoom command being indicated with respect to a first portion of a display in which first data from an electronic healthcare record, EHR is displayed;
ii) retrieve second data that is semantically linked to the first data; and
iii) in response to receiving the first user input, instruct the display to display the second data in a second portion of the display.

2. The apparatus of claim 1 wherein the processor being caused to retrieve the second data comprises the processor being caused to:
execute a computational function to retrieve the second data that is semantically linked to the first data.

3. The apparatus of claim 2 wherein the computational function uses natural language processing, NLP, or machine learning to retrieve the second data that is semantically linked to the first data.

4. The apparatus of claim 1 wherein the second data is logically connected to the first data as part of a hierarchical data structure in the EHR; and wherein the second data is in a neighbouring level of the hierarchical structure to the first data.

5. The apparatus as in any one of the preceding claims wherein: the second data is more detailed than the first data if the first user input comprises a zoom-in command and/or the second data is less detailed than the first data, if the first user input comprises a zoom-out command.

6. The apparatus as in any one of the preceding claims wherein the second portion of the display is at least partially overlapping with the first portion of the display and/or wherein the processor instructs the display to display the second data in place of the first data.

7. The apparatus as in any one of the preceding claims wherein the first portion of the display is in a first Graphical User Interface, GUI, the second portion of the display is in a second GUI and wherein the processor causes the second GUI to be initiated on the display in response to receiving the first user input.

8. The apparatus as in any one of the preceding claims wherein the set of instructions, when executed by the processor, further cause the processor to:
receive a second user input from the user input device, the second user input indicating a second zoom command with respect to the first portion of the display; and wherein the second zoom command at least partially reverses the first zoom command.

9. The apparatus of claim 8 wherein in response to receiving the second zoom command, the set of instructions, when executed by the processor, further cause the processor to: instruct the display to re-display the first data in the first portion of the display.

10. The apparatus of claim 8 wherein in response to receiving the second zoom command, the set of instructions, when executed by the processor, further cause the processor to:
retrieve third data that is semantically linked to the second data; and
instruct the display to display the third data in a third portion of the display; and
wherein the third data is different to the first data, if, since the processor performed step ii):
there has been a change in the first data;
there has been a change in the second data; or
the third data has become available; and
as a result, the third data is more highly semantically linked to the second data compared to the first data.

11. The apparatus as in any one of the preceding claims wherein:
the first data relates to a medical condition referred to in the EHR and the second data relates to a treatment given for the medical condition;
the first data relates to a medical condition referred to in the EHR and the second data relates to further information related to said medical condition; or
the first data relates to a type of medication referred to in the EHR and the second data relates to specific medications within said type.

12. An apparatus as in any one of the preceding claims wherein the first data is of a first data type and the second data is of a second data type that is different to the first data type.

13. A computer implemented method, the method comprising:
i) receiving a first user input from a user input device, the first user input indicating a first zoom command, the first zoom command being indicated with respect to a first portion of a display in which first data from an electronic healthcare record, EHR is displayed;
ii) retrieving second data that is semantically linked to the first data; and
iii) in response to receiving the first user input, instructing the display to display the second data in a second portion of the display.

14. A method as in claim 13 wherein the second data is more detailed than the first data if the first user input comprises a zoom-in command and/or the second data is less detailed than the first data, if the first user input comprises a zoom-out command.

15. A computer program product comprising a computer readable medium, the computer readable medium having computer readable code embodied therein, the computer readable code being configured such that, on execution by a suitable computer or processor, the computer or processor is caused to perform the method as claimed in claims 13 or 14.
